Europäisches Patentamt

European Patent Office

Office européen des brevets

(11) Publication number: **0 244 046**

**A1**

## (12) EUROPEAN PATENT APPLICATION

(21) Application number: **87300405.5**

(22) Date of filing: **19.01.87**

(51) Int. Cl.⁴: **A61N 1/42**

(30) Priority: **17.01.86 GB 8601138**

(43) Date of publication of application:
**04.11.87 Bulletin 87/45**

(84) Designated Contracting States:
**CH DE ES GB IT LI NL**

(71) Applicant: **Lyon, Ronald John**
**Rose Cottage High Street Stanton**
**Near Broadway Worcestershire(GB)**

(72) Inventor: **Lyon, Ronald John**
**Rose Cottage High Street Stanton**
**Near Broadway Worcestershire(GB)**

(74) Representative: **Burrows, Anthony Gregory**
**85 Pixmore Way**
**Letchworth Garden City Hertfordshire(GB)**

(54) **A therapy device.**

(57) A pulsed high-frequency electromagnetic energy therapy device includes a D.C. voltage generator 2 connectable to an A.C. mains supply. The generator 2 includes a pulse modulator 14 with a pulse frequency selector 15 and a pulse width selector 16. The modulator 14 emits a low-frequency low-voltage electrical signal which is conducted to a high-frequency oscillator circuit 7 the output of which is fed to an inductive electrode 6, the items 6 and 7 being contained in an applicator 3. The electrode 6 emits a pulsed high-frequency electromagnetic energy wave with a pulse mark/space ratio of more than 1:4.

FIG.5.

EP 0 244 046 A1

# A THERAPY DEVICE

This invention relates to a pulsed high-frequency electromagnetic energy therapy device.

GB1595121 discloses a pulsed high-frequency electromagnetic energy therapy device comprising a low-voltage power supply connected to a multivibrator producing electical pulses which are fed to a pulse shaper which is connected to a high-frequency stage itself connected to an antenna. A flexible applicator carries the antenna which is in the form of a double spiral the outer end of which is connected via a single conductor to the output of the high-frequency stage.The applicator includes a housing containing the multivibrator, the pulse shaper and the high-frequency stage. The low-voltage power supply is in another housing and may be a battery or a transformer. The output from the pulse shaper is connected additionally to a pair of low-frequency current-emitting electrodes. The pulse frequency of the output from the pulse shaper may be of the order of 1 Hertz to 10,000 Hertz, whilst the pulse width may be of the order of 10 to 100 microseconds. This gives a range of pulse mark/space ratios of from 1:100,000 to 1:0. A device according to this Patent is available and has pulse mark/space ratio of no more than 67:1,000, whereby the power emitted into a patient's tissue is relatively small and thus healing of the tissue is slow. Included in the multivibrator is an assembly which gives a non-adjustable pulse width. This feature restricts the adaptability of the device to the requirements of the physiotherapist.

GB2106394 discloses a device similar to that of GB1595121 with the major differences that it incorporates a reflector to reflect the outwardly moving part of the electromagnetic energy wave back towards the tissue and that the antenna is inductive rather than capacitive.

According to the present invention, there is provided a pulsed high-frequency electromagnetic energy therapy device, comprising wave-producing means for producing a pulsed high-frequency electrical wave, an electrode connected to the output of said means for transmitting a pulsed high-frequency electromagnetic energy wave, and an electrically insulating part covering a surface of said electrode and permeable by the latter wave for interposition between said electrode and a patient while in mechanical contact with, or immediately adjacent to, the skin of the patient, said means having a maximum rated power input of not more than 20 watts per electrode and said latter wave having a pulse frequency of between 1 and 2,000 Hertz, characterized in that said pulsed high-frequency electromagnetic energy wave has a pulse mark/space ratio of more than 1:4.

Owing to the present invention, it is possible to put greater energy into the tissue and thus promote relatively rapid healing, whilst retaining the advantages of low power operation, among which are its substantially athermic nature and its electrical safety.

The pulse frequency is pre-set or selectable and preferably between 1 and 800 Hertz and the mark/space ratio is pre-set or selectable and preferably more than 1:3 and may be less than 10:1.

Medical evidence shows that, for certain medical conditions, applying small pulsed electromagnetic fields to the part of the body affected has a healing effect, especially at relatively low pulse frequencies of between 1 and 800 Hertz. When electrodes in mechanical contact with the body, or immediately adjacent to the skin, i.e. no more than 2 cm from the skin, are used in conjunction with a relatively low power h.f. generator, it has been found highly beneficial to use a relatively high mark/space ratio of more than 1:4 so giving relatively long pulses which put more high-frequency energy into the tissue.

It is particularly advantageous for the wave-producing means to include pulse-width adjusting means, since this feature increases the adaptibility of the device to the physiotherapist's requirements.

It is also advantageous for at least the major part of the electrical circuitry to be separate from an applicator containing the electrode, since this makes the applicator more compact and easier to manipulate.

In order that the invention may be clearly understood and reaidly carried into effect, reference will now be made by way of example to the accompanying drawings, in which:-

Fig. 1 shows diagrammatically a readily portable, pulsed high-frequency electromagnetic energy therapy device,

Fig. 2 shows diagrammatically a perspective view of an applicator of the device,

Figs. 3A, 3B and 3C show diagrammatically front elevations and a longitudinal sectional view of the applicator with an electrode of an antenna character,

Fig. 4 illustrates diagrammatically various energy waves relative to the device,

Fig. 5 shows the device in more detail,

Fig. 6 shows diagrammatically a modified version of the device, and

Fig. 7 shows diagrammatically another modified version of the device.

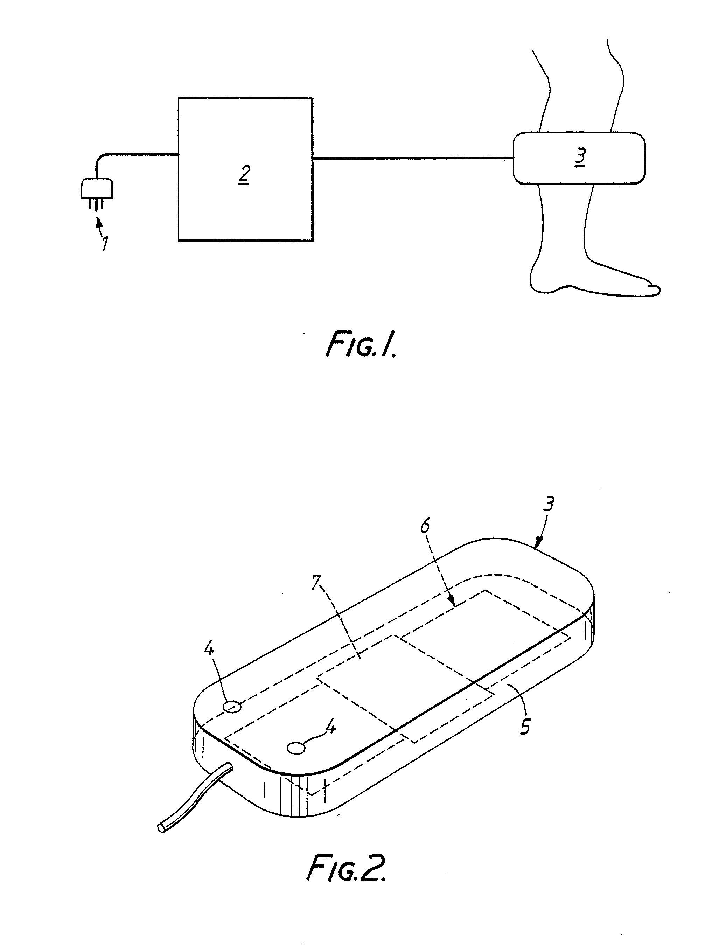

Referring to Fig. 1, an electrical mains supply plug 1 is connected to a generator 2 of pulsed D.C. voltage and continuous D.C. voltage (which can be any suitable value, but in this case 12 volts) the output of which is fed to an applicator 3 which is shown applied to the leg of the patient. The whole device can be contained easily in a conventional brief-case.

Referring to Fig. 2, the applicator 3 includes substantially rigid, electrically insulating housing 5 containing an h.f. oscillator circuit 7 connected to an electrode 6. The h.f. oscillator circuit includes a crystal controlling the frequency of the oscillator at 27.12 MHz and a power output stage connected to the electrode. The continuous D.C. voltage supply to the h.f. oscillator circuit causes it to oscillate continuously, whilst the pulsating D.C. voltage supply to the h.f. oscillator circuit switches the oscillator circuit on and off producing pulsed high-frequency current which is fed to the electrode 6 which emits pulsed high-frequency electromagnetic energy. A high-frequency energisation indicator lamp 4 is mounted on the housing 5 and connected to the power output stage of the circuit 7.

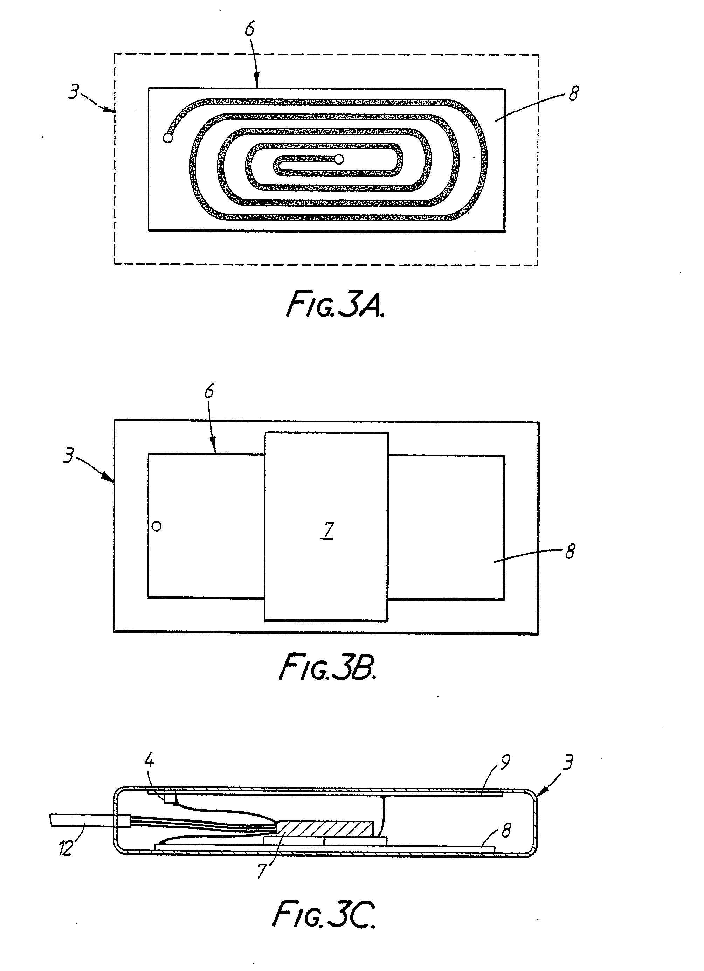

Referring to Figs. 3A, 3B and 3C, the electrode 6 is of a flat oblong spiral character and is formed by a printed circuit upon the front surface of a flat sheet of insulating material 8 which is fixed to the inside surface of the applicator front wall. The connection between the generator 2 and the applicator 3 is a three-core cable 12, whereof two cores are connected with the h.f. oscillator circuit 7, and the third core is connected to the power output stage of the circuit 7.

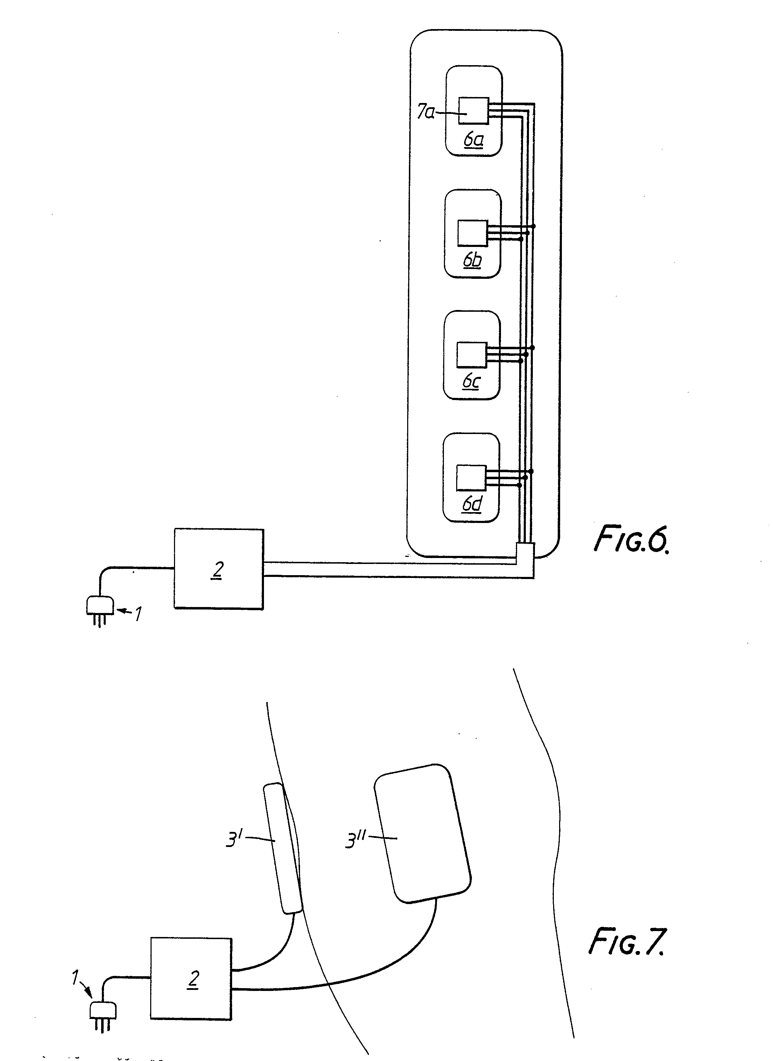

Fig. 5 shows that the generator 2 includes an A.C./D.C. converter 13, one output of which is connected to the h.f. oscillator in the circuit 7, via terminal 19, causing it to oscillate continuously, and the other output of which is connected to a pulse modulator 14 adjustable by a manually operable pulse frequency selector 15 and a manually operable pulse width selector 16. Alternatively, the pulse frequency and width may be fixed. The output from the pulse modulator 14 is pulsed D.C. voltage, which is fed to the power output stage of the circuit 7 in the applicator 3, via terminals 17 and 18. The oscillator ciruit 7 is switched on and off by the pulsating D.C. voltage, producing bursts of oscillations of 27.12 MHz and pulsed h.f. current of this frequency is fed to the electrode 6.

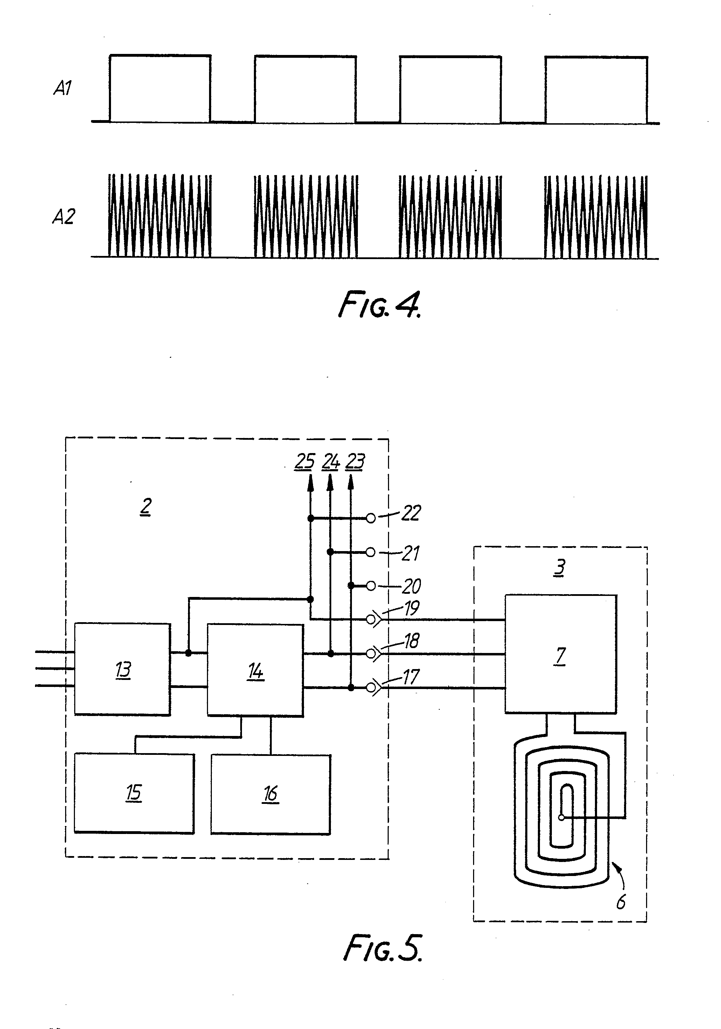

Fig. 4 illustrates at A1 the pulsed D.C. voltage output from the pulse modulator 14, and at A2 the bursts of h.f. oscillations from the oscillator circuit 7, the frequency and pulse width of the h.f. oscillations being the same as the frequency and pulse width of the D.C. voltage from the pulse modulator 14. The pulse frequency is between 1 and 2,000 Hz, preferably between 40 and 800 Hz, with an average mark/space ratio of at least 1:4, preferably between 1:3 and 10:1, for example about 1:1. Terminals 20, 21 and 22 are shown connected in parallel with the terminals 17, 18 and 19 respectively. A second applicator may be fed from the terminals 20 to 22; the pulsed h.f. electromagnetic waves from both applicators being of the same pulse frequency and pulse width and in synchronism with each other. Connections 23, 24 and 25 in parallel with terminals 20, 21 and 22 respectively are shown arrowed, indicating that any number of applicators may be supplied from the generator 2, all the outputs of which would in synchronism with each other and of the same pulse frequency and width. An applicator may instead contain several electrodes of an inductive character. All of the electrodes would be fed from a single generator with a single cable to the applicator, the electrodes being connected in parallel within the applicator to connections from the single connecting cable. For example, fig. 6 illustrates the output from the generator 2 supplied to an applicator including four electrodes 6a, 6b, 6c and 6d with a connecting cable from the generator 2 to the applicator 6. The power rating of the generator 2 is dependent on the number of electrodes to be supplied.

Fig. 7 illustrates the output from the generator 2 supplied to two applicators 3' and 3" arranged spaced about the patient's leg.

The present device is a low-power athermic device giving at the electrode 6 a power output not exceeding 200 mW/cm measured at the skin of the patient in the area being treated.

The device may comprise a main enclosure containing the A.C./D.C. converter and electronic circuitry, with a separate mains cable and plug or a built-in mains plug, the applicator containing the h.f. oscillator circuit(s) and the electrode(s) and being connected to the main enclosure by a flexible cable. Other possibilities include:

i) the main enclosure contains the converter only, with the electronic circuitry included in the applicator housing which is connected to the main enclosure by a flexible cable;

ii) the main enclosure contains the converter and all of the electronic circuitry including the h.f. oscillator circuit, the applicator containing the electrode being connected to the main enclosure by a low loss R.F. cable;

iii) the converter, all of the electronic circuitry and the electrode are included in a single enclosure. To give greater range of application, the device may incorporate batteries.

In any of the above versions of the device, the applicator and its electrode could take the form of:-

(a) An applicator housing with a rigid cover and with a flexible base to conform more easily to the body. The electrode could be a rigid printed circuit as in Fig. 3A fixed to the rigid applicator cover or a flexible printed circuit attached to the flexible base of the applicator, or

(b) A flexible printed circuit electrode contained within a flexible applicator.

Flexible electrodes may easily be provided using flexible printed circuit techniques to provide a similar spiral pattern to that shown in Fig 3A. The flexible applicator could take the form of an electrically insulating flexible plastics envelope permanently enclosing the electrode or a similar but replaceable envelope enclosing the electrode.

## Claims

1. A pulsed high-frequency electrogmagnetic energy therapy device, comprising wave-producing means (2,7) for producing a pulsed high-frequency electrical wave (A2), an electrode (6) connected to the output of said means (2,7) for transmitting a pulsed high-frequency electromagnetic energy wave, and an electrically insulating part (8) covering a surface of said electrode (6) and permeable by the latter wave for interposition between said electrode (6) and a patient while in mechanical contact with, or immediately adjacent to, the skin of the patient, said means (2,7) having a maximum rated power input of not more than 20 watts per electrode and said latter wave having a pulse frequency of between 1 and 2,000 Hertz, characterized in that said pulsed high-frequency electromagnetic energy wave has a pulse mark/space ratio of more than 1:4.

2. A device according to claim 1, wherein said means (2,7) includes pulse-width-adjusting means (16) for adjusting the pulse width.

3. A device according to claim 1 or 2, wherein said ratio is more than 1:3.

4. A device according to any preceding claim, wherein said ratio is less than 10:1.

5. A device according to any preceding claim, wherein said electrode (6) is connected to said wave-producing means (2,7) by way of an input conductor (17) and an output conductor (18), whereby said patient is subjected to a predominantly inductively produced field.

6. A device according to any preceding claim, wherein said electrode (6) and said electrically insulating part (8) are incorporated in an applicator (3), which also incorporates, of said wave-producing means (2,7) only a high-frequency oscillator circuit (7).

7. A device according to any one of claims 1 to 5, wherein said electrode (6) and said electrically insulating part (8) are incorporated in an applicator (3) and said electrode (6) is connected to a high-frequency oscillator circuit (7) of said means (2,7) by way of a flexible electrical conductor external of said applicator.

8. A device according to any preceding claim and further comprising at least a second electrode (6b) connected to said wave-producing means (2,7) for transmitting a second pulsed high-frequency electromagnetic energy wave, and at least a second electrically insulating part (8) covering a surface of said second electrode (6b) and permeable by this second wave.

9. A device according to claim 8, wherein the electrodes (6a,6b) and the electrically insulating parts (8) are incorporated in a common applicator (3).

10. A device according to claim 8, wherein the electrodes (6a,6b) and their respective electrically insulating parts (8) are incorporated in respective applicators (3',3").

FIG.1.

FIG.2.

FIG.3A.

FIG.3B.

FIG.3C.

FIG. 4.

FIG. 5.

7a

6a

6b

6c

6d

2

1

*FIG.6.*

3'

3''

2

1

*FIG.7.*

# EUROPEAN SEARCH REPORT

European Patent Office

Application number

EP 87 30 0405

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 371 205 (ELECTRO-BIOLOGY) * Page 4, line 37 - page 5, line 6; page 22, lines 34-38; page 24, lines 9-26; figure 5 * | 1-5,7, 9 | A 61 N 1/42 |
| A | WO-A-8 502 547 (GYÜLING) * Page 4, lines 3-10 * | 1-4 | |
| A | EP-A-0 104 793 (ELECTRO-BIOLOGY) * Page 6, line 2 - page 10, line 18; figures * | 1,5-9 | |

TECHNICAL FIELDS SEARCHED (Int. Cl.4)

A 61 N

The present search report has been drawn up for all claims

| Place of search | Date of completion of the search | Examiner |
|---|---|---|
| THE HAGUE | 29-04-1987 | LEMERCIER D.L.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82